# EUROPEAN PATENT APPLICATION

(11) **EP 4 772 029 A1**
(43) Date of publication of application: **08.07.2026**
(21) Application number: 24860112.2
(22) Date of filing: 25.06.2024
(51) Int. Cl.: A24B 15/16, A24B 13/00, A24F 23/02, A24B 15/24, A24B 15/30, A24B 15/38, A24B 15/32, A24B 15/42, A24B 15/28

(54) **FILLER FOR ORAL POUCH, AND ORAL POUCH COMPRISING SAME**

(30) Priority: 31.08.2023 KR 20230115720
(71) Applicant: KT&G Corporation, Daedeok-gu Daejeon 34337 (KR)
(72) Inventor: CHA, Sung Je, Daejeon 34128 (KR); KI, Sung Jong, Daejeon 34128 (KR); JEOUNG, Eunmi, Daejeon 34128 (KR); SHIN, Jun Won, Daejeon 34128 (KR)
(74) Representative: Ter Meer Steinmeister & Partner
(86) International application number: PCT/KR2024/008753
(87) International publication number: WO 2025/048188

(57) **Abstract**

The present invention relates to a filler, comprising an active material, for an oral pouch, the filler comprising large particles having a diameter of at least 400 um, medium particles having a diameter of 200 to less than 400 um, small particles having a diameter of 50 to less than 200 um, and fine particles having a diameter of less than 50 um, wherein the large particles are included at 20 wt% or less relative to the total weight of the filler, and the fine particles are included at 5 wt% or less relative to the total weight of the filler

## Description

### TECHNICAL FIELD

One or more embodiments relate to a filler for an oral pouch and an oral pouch including the filler.

### BACKGROUND ART

Oral pouches are pouches used in the user's mouth to allow the user to absorb active materials such as nicotine, and oral products in a form of pouches, such as snus, are manufactured and packaged in an appropriate form using granules containing active materials, including nicotine, using pouch packaging materials. Consumers may feel the satisfaction of the active materials for a predetermined period of time by placing the product in the form of a pouch between the gums and upper lip in the mouth. The elution characteristics of such active materials are the most essential characteristic of oral products and may be adjusted depending on the characteristics of a pouch material, but may be affected by a combination of fillers and a preparation method.

Therefore, research is required for more effective elution characteristics of active materials, including nicotine, from oral pouch fillers.

### DISCLOSURE OF THE INVENTION

### TECHNICAL GOALS

Embodiments provide a filler for an oral pouch with improved elution characteristics of an active material by adjusting a particle size and a mixing ratio of the filler.

However, goals to be achieved are not limited to those described above, and other goals not mentioned above are clearly understood by one of ordinary skill in the art from the following description.

### TECHNICAL SOLUTIONS

According to an aspect, there is provided a filler for an oral pouch, the filler including an active material, wherein the filler includes large particles with a diameter of 400 µm or more, medium particles with a diameter of 200 µm or more and less than 400 µm, small particles with a diameter of 50 µm or more and less than 200 µm, and fine particles with a diameter of less than 50 µm, the large particles are included in an amount of 20 wt% or less with respect to a total weight of the filler, and the fine particles are included in an amount of 5 wt% or less with respect to the total weight of the filler.

According to another aspect, there is provided an oral pouch including the filler for the oral pouch, and a packaging material that wraps the filler.

### EFFECTS OF THE INVENTION

When the present disclosure is used, it is possible to provide a filler for an oral pouch capable of allowing a consumer of the oral pouch to achieve a quick recognition and satisfaction of an active material during use due to excellent elution characteristic of the active material in the oral pouch, and capable of eluting most or all of an applied active material within a predetermined period of time (about 20 minutes).

It should be understood that the effects of the present disclosure are not limited to the above-described effects, but are construed as including all effects that may be inferred from the configurations and features described in the following description or claims of the present disclosure.

### BEST MODE FOR CARRYING OUT THE INVENTION

Hereinafter, embodiments will be described in detail with reference to the accompanying drawings. However, various alterations and modifications may be made to the embodiments. Here, the embodiments are not construed as limited to the disclosure. The embodiments should be understood to include all changes, equivalents, and replacements within the idea and the technical scope of the disclosure.

The terminology used herein is for the purpose of describing particular embodiments only and is not to be limiting of the embodiments. The singular forms "a", "an", and "the" are intended to include the plural forms as well, unless the context clearly indicates otherwise. It will be further understood that the terms "comprises/comprising" and/or "includes/including" when used herein, specify the presence of stated features, integers, steps, operations, elements, and/or components, but do not preclude the presence or addition of one or more other features, integers, steps, operations, elements, components and/or groups thereof.

Unless otherwise defined, all terms including technical and scientific terms used herein have the same meaning as commonly understood by one of ordinary skill in the art to which the embodiments belong. It will be further understood that terms, such as those defined in commonly-used dictionaries, should be interpreted as having a meaning that is consistent with their meaning in the context of the relevant art and will not be interpreted in an idealized or overly formal sense unless expressly so defined herein.

When describing the embodiments with reference to the accompanying drawings, like reference numerals refer to like components and a repeated description related thereto will be omitted. In the description of embodiments, detailed description of well-known related structures or functions will be omitted when it is deemed that such description will cause ambiguous interpretation of the present disclosure.

In addition, the terms first, second, A, B, (a), and (b) may be used to describe constituent elements of the embodiments. These terms are used only for the purpose of discriminating one component from another component, and the nature, the sequences, or the orders of the components are not limited by the terms.

A component, which has the same common function as a component included in any one embodiment, will be described by using the same name in other embodiments. Unless otherwise mentioned, the descriptions of an embodiment may be applicable to other embodiments and thus, repeated descriptions will be omitted for conciseness.

It will be understood that when a certain part "includes" a certain component, the part does not exclude another component but may further include another component.

According to an embodiment, there is provided a filler for an oral pouch including an active material, wherein the filler includes large particles with a diameter of 400 µm or more, medium particles with a diameter of 200 µm or more and less than 400 µm, small particles with a diameter of 50 µm or more and less than 200 µm, and fine particles with a diameter of less than 50 µm, the large particles are included in an amount of 20 wt% or less with respect to a total weight of the filler, and the fine particles are included in an amount of 5 wt% or less with respect to the total weight of the filler.

The filler for the oral pouch according to an embodiment may realize excellent elution characteristics of an active material existing in the pouch as the filler has the configuration described above.

Effective elution characteristics of a product in a pouch form refers to that, from the consumer's point of view, a quick recognition or satisfaction is required due to the elution of the active material during use, and at the same time, all applied active material must be eluted within a predetermined period of time. For example, for a product in a pouch form with a small size, it may be desirable that the size is 30 mm 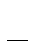 12 mm and a weight is 0.3 to 0.5 g, and the active material is eluted out during a usage time of approximately 20 minutes. More specifically, it may be desirable that an elution rate of about 20% within first 4 minutes and an elution rate of about 80% for 20 minutes are realized.

According to an embodiment, when a proportion of the large particles exceeds 20% in the particles included in the filler for the oral pouch, an initial moisture penetration effect is excellent, which may lead to a rapid elution effect of an active material such as nicotine, however, the elution rate over time may be delayed. Therefore, the proportion of the large particles may be desirably 20 wt% or less, more desirably 15 wt% or less with respect to a total weight of the filler.

Also, when the proportion of fine particles among the particles included in the filler exceeds 5 wt%, the initial moisture penetration effect is low, which may result in slow elution rate of the active material such as nicotine, but the elution rate increases over time. Therefore, the proportion of the fine particles may be desirably 5 wt% or less, more desirably 2 wt% or less with respect to the total weight of the filler.

Meanwhile, among the particles included in the filler, the content of medium particles may be greater than that of the small particles. In this case, the elution rate of the filler may be better than when the content of the small particles is greater than that of the medium particles.

In addition, an average particle size of the filler for the oral pouch according to an embodiment may be 150 to 400 µm, desirably 200 to 350 µm, and more desirably 250 to 300 µm. When the average particle size is less than the lower limit described above, manufacturing workability may be reduced due to a fine powder at a pouch packaging stage, and initial saliva may not be easily absorbed during use of the pouch. On the other hand, when the average particle size exceeds the upper limit described above, there may be a delay in elution of the active material such as nicotine inside the particles during use of the pouch.

The filler for the oral pouch according to an embodiment may include a bulking agent, a flavoring agent, a sweetener, a pH adjuster, a binder, and the like, along with the active material.

The active material is a material that may be absorbed through the oral mucosa or gums, and may include, specifically, nicotine, vitamin, caffeine, amino acid, a salt or a derivative thereof, and desirably, nicotine. The nicotine may be synthetic nicotine and/or a nicotine extract from tobacco plant.

Particularly, when a nicotine salt or the like is applied as the active material, the pH adjuster may be applied to generate free nicotine by providing a basic environment during product use, and specific examples of the pH adjuster may include metal hydroxide such as sodium hydroxide and potassium hydroxide, metal carbonate such as sodium carbonate and potassium carbonate, sodium bicarbonate, sodium sesquicarbonate (Na3H(CO3)2), and the like.

The bulking agent may include cellulose, sugar alcohol, a non-tobacco plant material, and the like, and specific examples may include cellulose includes microcrystalline cellulose (MCC), methylcellulose, hydroxypropyl cellulose (HPC), hydroxypropylmethyl cellulose (HPMC), hydroxyethyl cellulose, carboxymethyl cellulose, and the like. In addition, specific examples of the sugar alcohol may include isomalt, erythritol, sorbitol, arabitol, ribitol, maltitol, dulcitol, iditol, mannitol, xylitol, lactitol, and the like. Furthermore, examples of the non-tobacco plant material are not greatly limited and may include a coconut shell, a vegetable fiber, tea, herbs, coffee, fruits, and the like.

The flavoring agent may also be referred to as a "flavoring material", and refers to any aromatic substance that may change sensory properties associated with an oral product. The flavoring agent may be a natural flavoring agent or a synthetic flavoring agent, and specific types thereof may include vanilla, coffee, chocolate/cocoa, cream, mint, spearmint, menthol, peppermint, winter green, eucalyptus, lavender, cardamone, nutmeg, cinnamon, clove, cascarilla, Santalum album, honey, ginger, anise, sage, licorice, lemon, orange, apple, peach, lime, cherry, strawberry, and the like, but are not limited thereto.

The sweetener may be a natural sweetener or an artificial sweetener, and specific types thereof may include fructose, sucrose, glucose, maltose, mannose, galactose, lactose, stevia, honey, sucralose, isomaltose, maltodextrin, saccharin, aspartame, and the like, but are not limited thereto.

The binder may include one or more selected from a group consisting of HPC, HPMC, polyvinylpyrrolidone (PVP), sodium alginate, xanthan gum, guar gum, gellan gum, and gum arabic.

In addition, the filler for the oral pouch according to an embodiment may further include a humectant, an antioxidant, a preservative, and the like depending on the purpose and need of use, in addition to the composition described above.

According to an embodiment, an oral pouch including the filler for the oral pouch described in detail above, and a packaging material that wraps the filler may be provided.

The packaging material for the oral pouch is a material that is safe for the human body, may hold saliva well, has a predetermined strength to prevent tearing, and has a sealing property, and a material of the packaging material for the oral pouch may be a nonwoven type or woven type material, desirably a nonwoven type material. Unlike the woven type material, the non-woven type material has tangled fibers, and thus, there may be no vertical or horizontal direction, and cut edges may not unravel.

The packaging material for the oral pouch may specifically include cellulose fibers and thermoplastic material fibers.

At this time, the cellulose fibers may include not only cellulose but also a cellulose-based fiber, and the cellulose-based fiber may include at least one or more of a natural cellulose-based fiber or an artificial cellulose-based fiber.

In addition, the natural cellulose-based fiber may include at least one or more selected from a group consisting of a seed hair fiber, a bast fiber, a leaf fiber, and a wood fiber (pulp). The seed hair fiber may include, for example, cotton, bombax cotton, kapok, and the like. The bast fiber may include, for example, hemp, flax, jute, ramie grass, mulberry tree, and Edgeworthia chrysantha, and the like. The leaf fiber may include, for example, Manila hemp, New Zealand hemp, and the like. The wood fiber (pulp) may include at least one or more of softwood pulp or hardwood pulp, depending on the type and characteristics of the tree, and examples thereof may include a softwood fiber, a hardwood fiber, and the like.

The artificial cellulose-based fiber may include at least one or more of a regenerated cellulose fiber or a semi-synthetic fiber. The regenerated cellulose fiber may include, for example, viscose, rayon, forty acid, and the like. The semi-synthetic fiber may, for example, include an acetate fiber. The rayon may include not only rayon but also a rayon-based fiber that compensates for the shortcomings of rayon. The rayon-based fiber may include, for example, Tencel, lyocell, modal, and the like.

Meanwhile, the thermoplastic material fiber may include at least one or more selected from a group consisting of nylon, polyethylene, polylactic acid, polypropylene, polyethersulfone, and polyethylene terephthalate. By including the thermoplastic material fiber, the packaging material for the oral pouch may be sealed by heat.

Hereinafter, the present disclosure will be described in more detail with reference to examples, however, the present disclosure is not limited to the examples below.

### Examples

### 1. Preparation Example 1: Preparation of filler for oral pouch

A solid-phase filling material including nicotine salt, HPC as a binder, and MCC and maltitol as a filler, was prepared, and menthol, a peppermint oil, and alcohol were mixed to prepare a particle size control solution.

While stirring the prepared solid-phase filling material, the particle size control solution was sprayed onto the solid-phase filling material.

Then, after performing a granulation process by high-speed mixing, the material was dried at a low temperature (50°C) for 3 hours to obtain a filler.

The prepared filler was classified into large particles (400 µm or more), medium particles (200 µm or more and less than 400 µm), small particles (50 µm or more and less than 200 µm), and fine particles (less than 50 µm) to prepare a final filler for an oral pouch as in Examples 1 to 6 of Table 1 below.

### 2. Preparation Example 2: Preparation of packaging material for oral pouch and oral pouch

First, a cellulose fiber (viscose rayon, soft fiber) was beaten and then mixed with a thermoplastic material fiber (PE/PP) and water to prepare a dispersion. The dispersion was moved to a paper machine, dehydrated and dried to finally prepare a packaging material for an oral pouch including the cellulose fiber and the thermoplastic material fiber in a weight ratio of 6:4.

After that, 350 mg of the filler prepared in Preparation Example 1 was sealed with the packaging material (25 mm × 15 mm) to prepare a mini-sized oral pouch.

### 3. Experimental Example: Evaluation of elution characteristic

For the oral pouches of Examples 1 to 6 prepared in Preparation Example 2, the nicotine elution rate (%) was measured for each time period. The results thereof are shown in Table 1 below.

**[Table 1]**

| Classification | | Example 1 | Example 2 | Example 3 | Example 4 | Example 5 | Example 6 |
|---|---|---|---|---|---|---|---|
| Large particles (%) | | 3.1 | 30.7 | 1.8 | 1.1 | 34.5 | 14.4 |
| Medium particles (%) | | 32.1 | 63.3 | 39.4 | 40.5 | 51.5 | 55.8 |
| Small particles (%) | | 60.8 | 5.9 | 54.0 | 57.6 | 13.6 | 28.8 |
| Fine particles (%) | | 4.1 | 0.1 | 4.8 | 0.9 | 0.3 | 1.0 |
| Average particle size (µm) | | 177 | 354 | 189 | 194 | 360 | 274 |
| Elution nicotine (%) (cumulative) | 4 minutes | 13.9 | 16.4 | 11.2 | 16.3 | 18.7 | 19.2 |
| | 8 minutes | 34.5 | 37.1 | 28.7 | 38.9 | 37.6 | 41.9 |
| | 12 minutes | 52.0 | 51.5 | 45.2 | 55.9 | 51.3 | 57.6 |
| | 16 minutes | 63.8 | 61.2 | 57.8 | 67.8 | 61.4 | 71.0 |
| | 20 minutes | 70.2 | 67.7 | 66.8 | 76.5 | 68.5 | 80.8 |

When Examples 1 and 2 are compared, it may be confirmed that Example 1 has a high proportion of fine particles and a low average particle size, whereas Example 2 has a high proportion of large particles and a low proportion of fine particles. Through this, it may be confirmed that Example 2 has a high elution level at the beginning of use of the oral pouch, but nicotine elution over time tends to be lower than that of Example 1.

In addition, when Examples 3 and 4 are compared, it may be confirmed that the average particle size of the granules is similar, but the proportion of fine particles is higher in Example 3, and overall elution nicotine data is lower in Example 3 than in Example 4.

On the other hand, in Example 5, the proportion of large particles is high, and the nicotine elution level is high at the beginning of use, but it may be confirmed the elution rate is delayed over time.

When Examples 4 and 6, in which the proportion of large particles is 20 wt% or less and the proportion of fine particles is 5 wt% or less, are compared, it may be confirmed that, in Example 6, the proportion of medium particles is higher than the proportion of small particles, and the elution nicotine data shows that the elution level is most excellent, not only at the beginning of use but also over time.

While the embodiments are described with reference to drawings, it will be apparent to one of ordinary skill in the art that various alterations and modifications in form and details may be made in these embodiments without departing from the spirit and scope of the claims and their equivalents. For example, suitable results may be achieved if the described techniques are performed in a different order, and/or if components in a described system, architecture, device, or circuit are combined in a different manner, and/or replaced or supplemented by other components or their equivalents.

Therefore, other implementations, other embodiments, and equivalents to the claims are also within the scope of the following claims.

## Claims

1. A filler for an oral pouch comprising:
an active material,
wherein the filler comprises large particles with a diameter of 400 µm or more, medium particles with a diameter of 200 µm or more and less than 400 µm, small particles with a diameter of 50 µm or more and less than 200 µm, and fine particles with a diameter of less than 50 µm,
wherein the large particles are included in an amount of 20 wt% or less with respect to a total weight of the filler, and
wherein the fine particles are included in an amount of 5 wt% or less with respect to the total weight of the filler.

2. The filler of claim 1, wherein the large particles are included in an amount of 15 wt% or less with respect to the total weight of the filler.

3. The filler of claim 1, wherein the fine particles are included in an amount of 2 wt% or less with respect to the total weight of the filler.

4. The filler of claim 1, wherein a content of the medium particles is more than a content of the small particles.

5. The filler of claim 1, wherein the active material comprises one or more selected from a group consisting of nicotine, vitamin, caffeine, a salt thereof, and a derivative thereof.

6. The filler of claim 1, wherein the filler comprises one or more bulking agents of cellulose, sugar alcohol, and a non-tobacco plant material,
the cellulose comprises one or more selected from a group consisting of microcrystalline cellulose (MCC), methylcellulose, hydroxypropyl cellulose (HPC), hydroxypropylmethyl cellulose (HPMC), hydroxyethyl cellulose, and carboxymethyl cellulose, and
the sugar alcohol comprises one or more selected from a group consisting of isomalt, erythritol, sorbitol, arabitol, ribitol, maltitol, dulcitol, iditol, mannitol, xylitol, and lactitol.

7. The filler of claim 1, wherein the filler comprises one or more flavoring agents selected from a group consisting of vanilla, coffee, chocolate/cocoa, cream, mint, spearmint, menthol, peppermint, winter green, eucalyptus, lavender, cardamone, nutmeg, cinnamon, clove, cascarilla, Santalum album, honey, ginger, anise, sage, licorice, lemon, orange, apple, peach, lime, cherry, and strawberry.

8. The filler of claim 1, wherein the filler comprises one or more sweeteners selected from a group consisting of fructose, sucrose, glucose, maltose, mannose, galactose, lactose, stevia, honey, sucralose, isomaltose, maltodextrin, saccharin, and aspartame.

9. The filler of claim 1, wherein the filler comprises one or more pH adjusters selected from a group consisting of sodium hydroxide, potassium hydroxide, sodium carbonate, potassium carbonate, sodium bicarbonate, and sodium sesquicarbonate (Na3H(CO3)2).

10. The filler of claim 1, wherein the filler comprises one or more binders selected from a group consisting of HPC, HPMC, polyvinylpyrrolidone (PVP), sodium alginate, xanthan gum, guar gum, gellan gum, and gum arabic.

11. The filler of claim 1, wherein the filler further comprises one or more of a humectant, an antioxidant, and a preservative.

12. An oral pouch comprising the filler for the oral pouch of claim 1; and a packaging material that wraps the filler.
